# EUROPEAN PATENT SPECIFICATION

(11) **EP 2 528 948 B1**
(45) Date of publication and mention of the grant of the patent: **19.09.2018**
(21) Application number: 11737760.6
(22) Date of filing: 28.01.2011
(51) Int. Cl.: C07K 16/28, C12N 15/13, C12P 21/08, G01N 33/53, C07K 16/00

(54) **NOVEL LOWERED AFFINITY ANTIBODIES AND METHODS OF MAKING THE SAME**
NEUE ANTIKÖRPER MIT REDUZIERTER AFFINITÄT UND VERFAHREN ZU IHRER HERSTELUNG
NOUVEAUX ANTICORPS DE MOINDRE AFFINITÉ ET PROCÉDÉS DE FABRICATION ASSOCIÉS

(30) Priority: 28.01.2010 US 299162 P
(43) Date of publication of application: 05.12.2012
(73) Proprietor: AB Biosciences, Inc., Allston, MA 02134 (US)
(72) Inventor: CHANG, Hsiu-Ching, Lexington, MA 02420 (US)
(74) Representative: Bohmann, Armin K.
(86) International application number: PCT/US2011/022998
(87) International publication number: WO 2011/094593

(56) References cited:
- WO-A1-00/29584
- WO-A2-2010/088522
- WO-A2-2010/088522
- US-A- 4 595 661
- US-A1- 2003 219 845
- W. KUSHARYOTO ET AL: "Mapping of a hapten-binding site: molecular modeling and site-directed mutagenesis study of an anti-atrazine antibody", PROTEIN ENGINEERING DESIGN AND SELECTION, vol. 15, no. 3, 1 March 2002 (2002-03-01), pages 233-241, XP055065660, ISSN: 1741-0126, DOI: 10.1093/protein/15.3.233
- LIPPOW SHAUN M ET AL: "Computational design of antibody-affinity improvement beyond in vivo maturation", NATURE BIOTECHNOLOGY, NATURE PUBLISHING GROUP, NEW YORK, NY, US, vol. 25, no. 10, 1 October 2007 (2007-10-01), pages 1171-1176, XP002615650, ISSN: 1087-0156, DOI: 10.1038/NBT1336 [retrieved on 2007-09-23]
- KARPUSAS M ET AL: "Crystal Structure of the alpha1beta1 Integrin I Domain in Complex with an Antibody Fab Fragment", JOURNAL OF MOLECULAR BIOLOGY, ACADEMIC PRESS, UNITED KINGDOM, vol. 327, no. 5, 11 April 2003 (2003-04-11), pages 1031-1041, XP027101422, ISSN: 0022-2836, DOI: 10.1016/S0022-2836(03)00203-1 [retrieved on 2003-04-11]
- CLARK LOUIS A ET AL: "Affinity enhancement of an in vivo matured therapeutic antibody using structure-based computational design", PROTEIN SCIENCE, CAMBRIDGE UNIVERSITY PRESS, vol. 15, no. 5, 1 May 2006 (2006-05-01), pages 949-960, XP002548548, ISSN: 0961-8368, DOI: 10.1110/PS.052030506
- WINKLER K ET AL: "Changing the antigen binding specificity by single point mutations of an anti-p24 (HIV-1) antibody", THE JOURNAL OF IMMUNOLOGY, THE AMERICAN ASSOCIATION OF IMMUNOLOGISTS, US, vol. 165, no. 8, 15 October 2000 (2000-10-15), pages 4505-4514, XP002579393, ISSN: 0022-1767
- MCCARTHY B J ET AL: "Altering the fine specificity of an anti-Legionella single chain antibody by a single amino acid insertion", JOURNAL OF IMMUNOLOGICAL METHODS, ELSEVIER SCIENCE PUBLISHERS B.V.,AMSTERDAM, NL, vol. 251, no. 1-2, 1 May 2001 (2001-05-01) , pages 137-149, XP004233080, ISSN: 0022-1759, DOI: 10.1016/S0022-1759(00)00319-7
- R. RAFFAI ET AL: "Binding of an Antibody Mimetic of the Human Low Density Lipoprotein Receptor to Apolipoprotein E Is Governed through Electrostatic Forces. STUDIES USING SITE-DIRECTED MUTAGENESIS AND MOLECULAR MODELING", JOURNAL OF BIOLOGICAL CHEMISTRY, vol. 275, no. 10, 15 March 2000 (2000-03-15), pages 7109-7116, XP055065664, ISSN: 0021-9258, DOI: 10.1074/jbc.275.10.7109
- C. REID ET AL: "Structure activity relationships of monocyte chemoattractant proteins in complex with a blocking antibody", PROTEIN ENGINEERING DESIGN AND SELECTION, vol. 19, no. 7, 18 April 2006 (2006-04-18) , pages 317-324, XP055065666, ISSN: 1741-0126, DOI: 10.1093/protein/gzl015
- WRNKLER, K. ET AL.: 'Changing the antigen binding specificity by single point mutations of an Anti-p24 (HIV-1) antibody.' JOURNAL OF IMMUNOLOGY vol. 165, 15 October 2000, pages 4505 - 4514, XP002579393
- MACARTHY, B. J ET AL.: 'Altering the fine specificity of an anti-Legionella single chain antibody by a single amino acid insertion.' JOURNAL OF IMMUNOLOGICAL METHODS. vol. 251, 01 May 2001, pages 137 - 149, XP004233080

## Description

### FIELD OF THE INVENTION

The present invention is related to a method for producing a recombinant antibody with reduced binding affinity.

### BACKGROUND OF THE INVENTION

Because of their ability to bind an antigen with a high degree of specificity, monoclonal antibodies are widely used as research, diagnostic, and therapeutic reagents. In addition to their specific binding to an antigen, monoclonal antibodies may activate the complement system and effector cells through their Fc region. In order to properly interpret an antibody's biological properties, proper controls are essential. Without proper controls, it is difficult to establish a causal relationship between an antibody's specific binding activity and the biochemical and biological effects of the antibody.

Control monoclonal antibodies currently in use include: 1. antibodies secreted by naturally occurring plasmacytoma, with no known target antigens; 2. antibodies raised against antigens from evolutionarily distant species, such as KLH (keyhole limpet hemocyanin); 3. antibodies reactive with a known target antigen that is distinct from the antigen of interest.

In each of these cases, the control antibodies used have a poorly defined variable domain and uncertain antigen specificities. And in the third case, there remains the issue of cross reactivity. Because of this, it is not uncommon to encounter problems such as cross reactivity or non-specific binding when currently available control antibodies are used. Furthermore, due to the lack of an ideal control antibody, research is often performed using formulation vehicles, such as normal saline, as a control. In such cases, it is difficult, if not impossible, to distinguish whether an observed biochemical and/or biological effect is a direct result of the specific antigen/antibody interaction, or a result of nonspecific effects, such as interactions and biological effects of other parts of the antibody molecule or contaminants present in the antibody preparation, such as the host cell proteins. For at least these reasons, there is currently a great need for improved, rationally designed control antibodies.

WO 2010/088522 A2 is related to rationally designed lowered affinity antibodies for use in various *in vivo* and *in vitro* applications.

WO 00/29584 is related to antibody variants of parent antibodies which have one or more amino acids inserted into a hypervariable region of the parent antibody and a binding affinity for a target antigen which is at least about two-fold stronger than the binding affinity of the parent antibody for the antigen.

Kusharyoto W et al. (Kusharyoto W et al., Protein Engineering Design and Selection, vol. 15, no. 3, 2002-03-01, pages 233-241) report on the mapping of a hapten-binding site of an anti-atrazine antibody using molecular modelling and a site-directed mutagenesis study.

Lippow S M et al. (Lippow S M et al., Nature Biotechnology, vol. 25, no. 10, 2007-10-01, pages 1171-1176) report on computational design of antibody-affinity improvement beyond in vivo maturation.

Karpusas M et al. (Karpusas M et al., Journal of Molecular Biol, vol. 327, no. 5, 2003-04-11, pages 1031-1041) report on crystal structure of the alpha 1 beta 1 integrin I domain in complex with an antibody Fab fragment.

Clark L A et al. (Clark L A et al., Protein Science, vol. 15, no. 5, 01-05-2006, pages 949-960) report on affinity enhancement of an *in vivo* matured therapeutic antibody using structure-based computational design.

Winkler K et al (Winkler K et al., The Journal of Immunology, vol. 165, no. 8, 2000-10-15, pages 4505-4514) report on changing the antigen binding specificity by single point mutations of an anti-p24 (HIV-1) antibody.

McCarthy B J et al. (McCarthy B J et al., Journal of Immunological Methods, vol. 251, no. 1-2, 2001-05-01, pages 137-149) report on altering the fine specificity of an anti-Legionella single chain antibody by a single amino acid insertion.

Raffai R et al. (Raffai R et al., Journal of Biological Chemistry, vol. 275, no. 10, 2000-03-15, pages 7109-7116) report that binding of an antibody mimetic of the human low density lipoprotein receptor to apolipoprotein E is governed through electrostatic forces as shown by site-directed mutagenesis and molecular modelling.

Reid C et al. (Reid C et al., Protein Engineering Design and Selection, vol. 19, no. 7, 2006-04-18, pages 317-324) report on structure activity relationships of monocyte chemoattractant proteins in complex with a blocking antibody.

### SUMMARY OF THE INVENTION

The problem underlying the present invention is solved by the subject matter of the attached independent claim; preferred embodiments may be taken from the attached depending claims.

More specifically, the problem underlying the present invention is solved by a method for producing a recombinant antibody with reduced binding affinity, comprising the steps of:
a) identifying by contact frequency statistics at least one amino acid from within a complementary determining region (CDR) of an antibody which is capable of a binding interaction with a target antigen;
b) altering the amino acid sequence of the CDR by replacing at least one interacting amino acid with a non-interacting amino acid without altering the overall antibody structure; and
c) confirming the binding interactions with the antigen and the recombinant antibody are reduced or eliminated such that the recombinant antibody has an approximate dissociation constant (KD) of greater than or equal to about 10⁻⁷ M.

In an embodiment, the method further comprises
d) confirming the recombinant antibody has an *in vivo* half-life similar to endogenous circulating immunoglobulins.

In an embodiment, the identified amino acid in the CDR polypeptide sequence is selected from the group consisting of tyrosine, serine, asparagine, aspartic acid, tryptophan and arginine.

In an embodiment, said non-interacting amino acid is selected from the group consisting of alanine, valine, leucine, isoleucine, proline and methionine.

In an embodiment, said non- interacting amino acid is alanine.

In an embodiment, the step of identifying the interacting amino acid further includes analyzing a crystal structure depicting a complex between the antibody, antibody fragment, or the CDR polypeptide sequence and the antigen.

In an embodiment, the method further comprises the step of validating a loss of antigen binding.

In an embodiment, the step of validation is accomplished by FACS, ELISA, or radioimmunoassay.

In an embodiment, the method further comprises expressing the recombinant antibody in a mammalian cell system.

Disclosed is a method for producing a complementarity determining region (CDR) with reduced binding capability comprising the steps of (a) identifying from within a CDR polypeptide, at least one binding amino acid, which is capable of an interaction with an antigen; and (b) altering the at least one binding amino acid within the CDR polypeptide so that the resulting CDR has a reduced ability in binding the antigen. The CDR may be located within an antibody or antibody fragment. The method may also include the step of validating a loss of antigen binding. The validation step may be accomplished by FACS analysis.

In an embodiment of the invention as defined in the claims, the interaction is a hydrogen bond, a salt bridge, and/or a Van der Waal's force. In certain embodiments of the invention as defined in the claims, the binding amino acid is aspartic acid, glutamic acid, lysine, arginine, serine, threonine, tyrosine, asparagine, histidine or glutamine. In other embodiments of the invention as defined in the claims the binding amino acid is tyrosine, serine, asparagine, aspartic acid, tryptophan, arginine, phenylalanine or glycine. In some embodiments of the invention as defined in the claims the binding amino acid is replaced with a non-binding amino acid. In some embodiments of the invention as defined the in the claims non-binding amino acid is alanine, valine, leucine, isoleucine, proline or methionine.

The disclosure includes the antibody or antibody fragment generated using the methods of the invention. The antibody or antibody fragment may have a dissociation constant (K_{D}) of greater than or equal to about 10⁻⁷. The antibody or antibody fragment of the invention as defined in the claims may be a whole immunoglobulin molecules, a scFv, an Fab fragment, an Fab' fragment, an F(ab')2, an Fd fragment, an Fv or a disulfide linked Fv.

### BRIEF DESCRIPTION OF THE DRAWINGS

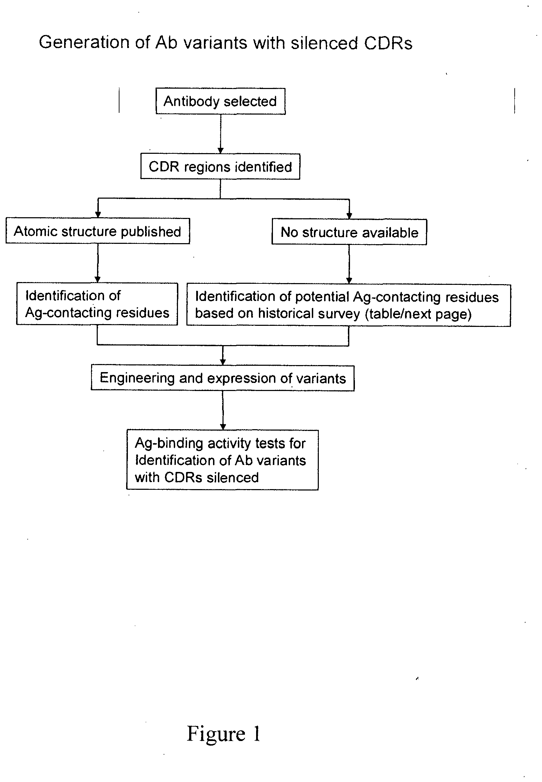

**Figure 1** shows a flow chart depicting the making of control antibodies of the invention.
**Figure 2** shows the frequency that specific CDR amino acids are involved in antibody-antigen interactions.
**Figure 3** shows an exemplary schematic diagram of an antibody structure, including the Fv domain, the Fab domain and the Fc domain.
**Figure 4** shows a schematic representation of an exemplary method of recombinant antibody production.
**Figure 5** shows antibody production of OKT3 and some exemplary control antibodies of the invention.
**Figure 6** shows FACS plots demonstrating binding of some exemplary control antibodies of the invention to human Jurkat cells.
**Figure 7** shows FACS plots demonstrating binding of some exemplary control antibodies of the invention to human PBMC.
**Figure 8** shows a map of the pME-wtOKT3 HC vector that encodes the OKT3 heavy chain.
**Figure 9** shows a map of the pME-wtOKT3 LC vector that encodes the OKT3 light chain.

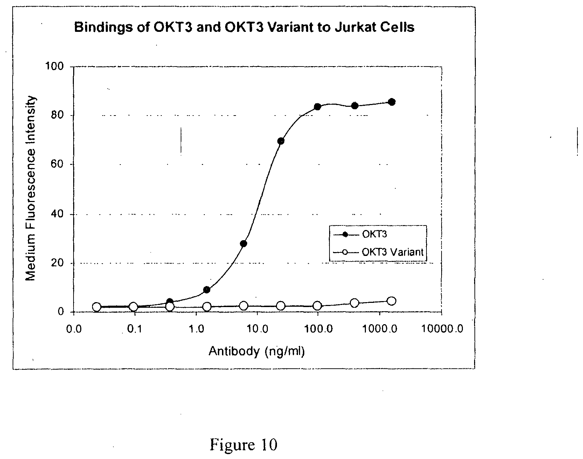

**Figure 10** shows the binding of OKT3 and OKT3 variant to Jurkat cells.

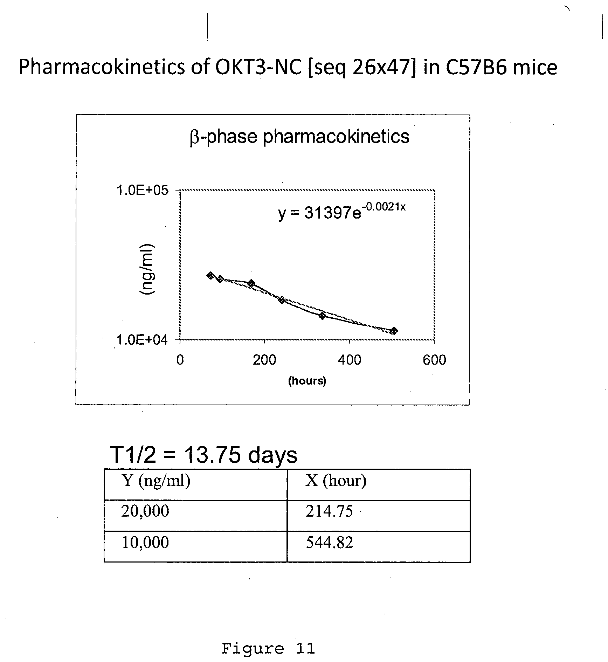

**Figure 11** shows a pharmacokinetics of the variant antibody.

### DETAILED DESCRIPTION OF THE INVENTION

The term "antibody" is well understood in biological and biomedical field and commonly refers to whole antibodies and any antibody fragment, or single chain thereof. Antibodies are glycoproteins secreted by specialized B lymphocytes known as plasma cells. They are also referred to as immunogobulins (Ig) because they contain a common structural domain found in many proteins. Antibodies most likely comprise two heavy (H) chains and two light (L) chains typically connected by disulfide bonds, or an antigen binding portion thereof. Each heavy chain is comprised of a heavy chain variable region (V_{H}) and a heavy chain constant region. Each light chain is also comprised of a variable region (V_{L}) and a constant region. The light chain constant region is comprised of one domain, CL. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen.

The present invention includes antibody fragments as well. Examples of antibody fragments include (i) a Fab fragment, a monovalent fragment consisting of the V_{H}, V_{L}, CL and CH1 domains; (ii) a F(ab')₂ fragment, a bivalent fragment comprising two Fab fragments linked by disulfide bridges at the hinge region; (iii) a Fd fragment consisting of the V_{H} and CH1 domains; (iv) a Fv fragment consisting of the V_{H} and V_{L} domains of a single arm of an antibody, (v) a dAb fragment (Ward et al., (1989) Nature 341:544 546), which consists of a V_{H} domain; and (vi) an isolated complementarity determining region (CDR) or (vii) a combination of two or more isolated CDRs which may optionally be joined by a synthetic linker. Furthermore, although the two domains of the Fv fragment, V_{H} and V_{L}, are coded for by separate genes, they can be joined, using recombinant methods, by a synthetic linker that enables them to be made as a single protein chain in which the V_{H} and V_{L} regions pair to form monovalent molecules (known as single chain Fv (scFv); see *e.g.,* Bird et al. (1988) Science 242:423 426; and Huston et al. (1988) Proc. Natl. Acad. Sci. USA 85:5879 5883). Such single chain antibodies are also intended to be encompassed within the term "antigen-binding portion" of an antibody. As used herein, "antibody" and "antibody fragments" are used interchangeably to describe the invention unless specifically stated otherwise.

The term *"hypervariable region," "HVR,"* or *"HV,"* refers to the regions of an antibody-variable domain that are hypervariable in sequence and form structurally defined loops. Generally, antibodies comprise six HVRs; three in the VH (H1, H2, H3), and three in the VL (L1, L2, L3). In an antibody molecule, the three HVRs of a VH domain and the three HVRs of a VL domain are brought together in three-dimensional structure to form an antigen binding surface. Because these sequences form a surface that is complementary to the three dimensional structure of the target antigen, the HVRs are also known as complementarity-determining regions (CDRs).

The terms "CDR", and its plural "CDRs", refer to a complementarity determining region (CDR) of which three make up the binding character of a light chain variable region (CDRL1, CDRL2 and CDRL3) and three make up the binding character of a heavy chain variable region (CDRH1, CDRH2 and CDRH3). CDRs contribute to the functional activity of an antibody molecule and are separated by amino acid sequences that comprise scaffolding or framework regions. The exact definitional CDR boundaries and lengths are subject to different classification and numbering systems. CDRs may therefore be referred to by Kabat, Chothia, contact or any other boundary definitions. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat, Chothia, and/or MacCallum et al., (Kabat et al., in "Sequences of Proteins of Immunological Interest," 5th Edition, U.S. Department of Health and Human Services, 1992; Chothia et al., J. Mol. Biol., 1987, 196: 901; and MacCallum et al., J. Mol. Biol., 1996, 262: 732).

"Framework region" or "FR" residues are those variable-domain residues other than the HVR residues as herein defined. Exemplary framework regions are provided as SEQ ID NOs. 15-22.

The term "Fc region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies of the invention include IgG, IgM, IgA, IgD and IgE.

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the Fc gamma RI, Fc gamma RII, and Fc gamma RIII subclasses, including allelic variants and alternatively spliced forms of these receptors, Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see M. Daëron, Annu. Rev. Immunol. 15:203-234 (1997). FcRs are reviewed in Ravetch and Kinet, Annu. Rev. Immunol. 9: 457-92 (1991); Capel et al., Immunomethods 4: 25-34 (1994); and de Haas et al., J. Lab. Clin. Med. 126: 330-41 (1995). Other FcRs, including those binding to other isotypes as well as those to be identified in the future, are encompassed by the term "FcR" herein.

Antibodies may be xenogeneic, allogeneic, or syngeneic; or modified forms thereof (*e.g*., humanized, chimeric, etc.). Antibodies may also be fully human. The term "monoclonal antibody" as used herein, refers to an antibody which displays a single binding specificity and affinity for a particular epitope. Accordingly, the term "human monoclonal antibody" refers to an antibody which displays a single binding specificity and which has variable and constant regions derived from human germline or non-germline immunoglobulin sequences. In one embodiment, human monoclonal antibodies are produced by a hybridoma which includes a B cell obtained from a transgenic non-human animal, e.g., a transgenic mouse, having a genome comprising human heavy and light chain transgenes fused to an immortalized cell.

In some embodiments of the invention as defined in the claims, antibodies, or fragments thereof, are modified to reduce or eliminate potential glycosylation sites. Such modified antibodies are often referred to as *"aglycosylated"* antibodies. In order to improve the binding affinity of an antibody or antigen-binding fragment thereof, glycosylation sites of the antibody can be altered, for example, by mutagenesis (*e.g.,* site-directed mutagenesis). *"Glycosylation sites"* refer to amino acid residues which are recognized by a eukaryotic cell as locations for the attachment of sugar residues. The amino acids where carbohydrate, such as oligosaccharide, is attached are typically asparagine (N-linkage), serine (O-linkage), and threonine (O-linkage) residues. In order to identify potential glycosylation sites within an antibody or antigen-binding fragment, the sequence of the antibody is examined, for example, by using publicly available databases such as the website provided by the Center for Biological Sequence Analysis (see http://www.cbs.dtu.dk/services/NetNGlyc/ for predicting N-linked glycosylation sites) and http://www.cbs.dtu.dk/services/NetOGlyc/ for predicting O-linked glycosylation sites). Additional methods for altering glycosylation sites of antibodies are described in U.S. Pat. Nos. 6,350,861 and 5,714,350. Further, antibody glycosylation can be influenced by the cell in which it is produced, the conformation of an antibody and the cell culture conditions. The preferred cell expression system of the invention is human cell expression system.

The term *"humanized antibody"* refers to an antibody that consists of the CDR of antibodies derived from mammals other than human, and the FR region and the constant region of a human antibody. A humanized antibody is useful as an effective component in a therapeutic agent since antigenicity of the humanized antibody in human body is lowered. It is an objective of the present invention to design lowered affinity antibodies to humanized antibodies in the context of therapeutic applications.

The term *"recombinant antibody"* includes all antibodies that are prepared, expressed, created or isolated by recombinant means, such as (a) antibodies isolated from an animal (e.g., a mouse) that is transgenic or transchromosomal for immunoglobulin genes or a hybridoma prepared therefrom (described further in Section I, below), (b) antibodies isolated from a host cell transformed to express the antibody, *e.g*., from a transfectoma, (c) antibodies isolated from a recombinant, combinatorial antibody library, and (d) antibodies prepared, expressed, created or isolated by any other means that involve splicing of immunoglobulin gene sequences to other DNA sequences. Such recombinant antibodies have variable and constant regions derived from germline and/or non-germline immunoglobulin sequences. In certain embodiments, however, such recombinant antibodies can be subjected to *in vitro* mutagenesis (or, when an animal transgenic for Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the germline repertoire *in vivo.*

The term "bispecific monoclonal antibody" refers to a monoclonal antibody having dual specificity in their binding arms to two different types of antigen. Bispecific monoclonal antibodies do not occur naturally; they have to be made with recombinant DNA or cell fusion technology. With this approach, it is possible for such an antibody to simultaneously bind to a cytotoxic cell (using a receptor like CD3) and a cancer target cell (like CD19), leading to more efficient killing of target cancer cells. It is the objective of the present invention to design lowered affinity antibodies to bispecific monoclonal antibodies such that one or both arms of the biospecific antibody will have reduced or eliminated affinity to an antigen.

The term "K_{D}" is intended to refer to the dissociation equilibrium constant of a particular antibody-antigen interaction. The measure of the binding strength of an antibody for a monovalent epitope is referred to as affinity. In some instances, antibodies can form multivalent interactions with antigen. In such cases, the apparent dissociation equilibrium constant of an antibody/antigen interaction may vary from the monovalent dissociation constant.

Affinity varies depending on the non-covalent bonds that exist between the antigen combining site on the antibody and the antigenic determinant or epitope of the specific antigen. In a typical situation, antibodies are used for their specific binding properties and the antibody binds with an affinity (K_{D}) of approximately less than 10⁻⁷ M, such as approximately less than 10⁻⁸ M, 10⁻⁹ M or 10⁻¹⁰ M or even lower when determined, for example, by surface plasmon resonance (SPR) technology in a BIACORE instrument using recombinant proteins as the analyte and the antibody as the ligand, and binds to the predetermined antigen with an affinity that is at least 1.1-, 1.2-, 1.3-, 1.4-, 1.5-, 1.6-, 1.7-, 1.8-, 1.9-, 2.0-, 2.5-, 3.0-, 3.5-, 4.0-, 4.5-, 5.0-, 6.0-, 7.0-, 8.0-, 9.0-, or 10.0-fold or greater than its affinity for binding to a non-specific antigen (e.g., BSA, casein) other than the predetermined antigen or a closely-related antigen. The phrases "an antibody recognizing an antigen" and "an antibody specific for an antigen" are used interchangeably herein with the term "an antibody which binds specifically to an antigen". An antibody that has "lowered affinity" refers to a K_{D} of approximately greater than 10⁻⁷ M, such as approximately 10, or 100, or 1000 fold greater than 10⁻⁷ M. It is the objective of the present invention to design an antibody with little or no specific binding to an antigen. In some embodiments the antibodies of the invention have a dissociation constant (K_{D}) of greater than or equal to about 10⁻⁷. More preferably, the antibodies obtained by a method of the invention as defined in the claims have a dissociation constant (K_{D}) of greater than or equal to about 10⁻⁶. Most preferably, the antibodies obtained by a method of the invention as defined in the claims have no detectable specific binding towards a defined antigen.

Depending on the Ig class, up to five structural molecules may be combined to form any one antibody. In mammals, there are five classes of Ig (IgG, IgM, IgA, IgD, and IgE); and in avians, there are three classes (IgY, IgM, and IgE). In select mammanls, IgG and IgA are further subdivided into subclasses, referred to as isotypes, due to polymorphisms in the conserved regions of the heavy chain.

The term "nucleic acid molecule" or "polynucleotide" is intended to include DNA molecules and RNA molecules. A nucleic acid molecule may be single-stranded or double-stranded, but preferably is double-stranded DNA.

The methods of the invention as defined in the claims also encompasses "conservative sequence modifications" of the sequences set forth in the figures, including nucleotide and amino acid sequence modifications which do not significantly affect or alter the binding characteristics of the antibody encoded by the nucleotide sequence or containing the amino acid sequence. Such conservative sequence modifications include nucleotide and amino acid substitutions, additions and deletions. Modifications can be introduced into the sequence set forth in the figures by standard techniques known in the art, such as site-directed mutagenesis and PCR-mediated mutagenesis. Conservative amino acid substitutions include ones in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g.*, aspartic acid, glutamic acid), uncharged polar side chains (*e.g.*, glycine, asparagine, glutamine, serine, threonine, tyrosine, cysteine, tryptophan), nonpolar side chains (*e.g.*, alanine, valine, leucine, isoleucine, proline, phenylalanine, methionine), beta-branched side chains (*e.g.,* threonine, valine, isoleucine) and aromatic side chains (*e.g.,* tyrosine, phenylalanine, tryptophan, histidine).

Accordingly, antibodies encoded by the heavy and light chain variable region nucleotide sequences disclosed herein and/or containing the heavy and light chain variable region amino acid sequences disclosed herein include substantially similar antibodies encoded by or containing similar sequences which have been conservatively modified. Further discussion as to how such substantially similar antibodies can be generated based on the sequences (*i.e.,* heavy and light chain variable regions) disclosed herein is provided below.

The nucleic acid compositions of the present disclosure may be mutated, in accordance with standard techniques to provide gene sequences. For coding sequences, these mutations, may affect amino acid sequence as desired. In particular, DNA sequences substantially homologous to or derived from native V, D, J, constant, switches and other such sequences described herein are contemplated (where "derived" indicates that a sequence is identical or modified from another sequence).

Various aspects and embodiments of the invention as defined in the claims are described in further detail in the following subsections.

### I. Lowered Affinity Antibodies

Lowered affinity antibodies obtained by a method of the present invention as defined in the claims are antibodies that were rationally designed to eliminate or reduce binding to an antigen. A currently preferred lowered affinity antibody is one that resembles the wild type (native) antibody in every respect except for the CDR region and shows little discernable 3 dimensional structural change relative to the wild type antibody. An exemplary antibody is one that demonstrates a decreased 5):as defined in the claims binding affinity to an antigen by 10 fold. Preferably, a lowered affinity antibody binds to an antigen with a decreased binding affinity to an antigen by 100 fold. More preferably, a lowered affinity antibody binds to an antigen with a decreased binding affinity to an antigen by 1000 fold. Most preferably, a lowered affinity antibody shows no substantial binding to an antigen, and no specific binding to an antigen is detectible over the background binding levels. The affinity can be measured by common experimental methods including surface Plasmon resonance, or radio ligand binding assay, or flow cytometry. Lowered affinity antibodies of the present invention are useful as control reagents for their wild type (native) counterpart that retains specific binding to an antigen in many in vivo and in vitro applications that involve the use of antibodies.

Lowered affinity antibodies can be produced using a variety of known techniques, such as the recombinant DNA techniques and other standard molecular and cell biology techniques.

Recombinant lowered affinity antibodies can be made using recombinant DNA techniques and gene trarisfection methods well known in the art (Morrison, S. (1985) Science 229: 1202). For example coding sequences, antibody-encoding polynucleotides with known antigen specificity can be amplified by standard molecular biology techniques (e.g. polymerase chain reaction) and ligated into an expression vector (e.g. pME). Mutations can be introduced into the expression vector using standard site directed mutagenesis techniques in order to disrupt antigen binding of the encoded antibodies. Expression vectors encoding the mutated antibody heavy and light chain genes can be transfected into host cells (e.g. 293T cells, CHO-cells) using techniques known in the art (e.g. calcium phosphate precipitation, electroporation, lipofection).

Following introduction of the expression vectors into the host cells, cells will begin to produce and secret mutated antibodies into the culture media. Long term, large scale antibody production can be achieved, for example, by identifying and selecting cells expressing the mutated antibody. Recombinant antibodies can be isolated and purified from these culture supernatants and/or cells using techniques known in the art. Alternatively, mutated antibodies can be produced in other expression systems or complete organisms or can be produced synthetically.

In another embodiment lowered affinity antibodies can be generated as chimeric antibodies in which the variable regions of the antibody heavy and/or light chains are fused to constant domains from various species.

In addition, humanized lowered affinity antibodies can be made according to standard protocols such as those disclosed in US patent 5,565,332. In another embodiment, antibody chains can be produced by recombination between vectors comprising nucleic acid molecules encoding a fusion of a polypeptide chain of a specific antibody chain and a component of a replicable generic display package and vectors containing nucleic acid molecules encoding a second polypeptide chain of a single binding pair member using techniques known in the art, e.g., as described in US patents 5,565,332, 5,871,907, or 5,733,743.

Lowered affinity antibodies can be produced using recombinant DNA techniques known in the art, for example using methods described in Robinson et al. International Patent Publication WO 87/02671; Akira et al. European Patent Application 184,187; Taniguchi, M. European Patent Application 171,496; Morrison et al. European Patent Application 173,494; Neuberger et al. PCT Application WO 86/01533; Cabilly et al. U.S. Patent No. 4,816,567; Cabilly et al. European Patent Application 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. 84:214-218; Nishimura et al. (1987) Cancer Res. 47:999-1005; Wood et al. (1985) Nature 314:446-449; and Shaw et al. (1988) J. Natl. Cancer Inst. 80:1553-1559); Morrison, S. L. (1985) Science 229:1202-1207; Oi et al. (1986) Biotechniques 4:214; Winter U.S. Patent 5,225,539; Jones et al. (1986) Nature 321:552-525; Verhoeyan et al. (1988) Science 239:1534; and Beidler et al. (1988) J. Immunol. 141:4053-4060.

In still another aspect of the invention as defined in the claims, partial or known antibody sequences can be used to generate and/or express lowered affinity antibodies. Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of a specific antibody by constructing expression vectors that include CDR sequences from the specific antibody grafted onto framework sequences from a different antibody with different properties (see, e.g., Riechmann, L. et al., 1998, Nature 332:323-327; Jones, P. et al., 1986, Nature 321:522-525; and Queen, C. et al., 1989, Proc. Natl. Acad. See. U.S.A. 86:10029-10033). Such framework sequences can be obtained from public DNA databases that include germline or non-germline antibody gene sequences. These germline sequences will differ from mature antibody gene sequences because they will not include completely assembled variable genes, which are formed by V(D)J joining during B cell maturation. Germline gene sequences will also differ from the sequences of a high affinity secondary repertoire antibody at individual evenly across the variable region. For example, somatic mutations are relatively infrequent in the amino-terminal portion of framework region. For example, somatic mutations are relatively infrequent in the amino terminal portion of framework region 1 and in the carboxy-terminal portion of framework region 4. Furthermore, many somatic mutations do not significantly alter the binding properties of the antibody. For this reason, it is not necessary to obtain the entire DNA sequence of a particular antibody in order to recreate an intact recombinant antibody having binding properties similar to those of the original antibody (see WO 1999/045962 filed on Mar. 12, 1999). Partial heavy and light chain sequence spanning the CDR regions is typically sufficient for this purpose. The partial sequence is used to determine which germline and/or non-germline variable and joining gene segments contributed to the recombined antibody variable genes. The germline and/or non-germline sequence is then used to fill in missing portions of the variable regions. Heavy and light chain leader sequences are cleaved during protein maturation and do not contribute to the properties of the final antibody. To add missing sequences, cloned cDNA sequences can be combined with synthetic oligonucleotides by ligation or PCR amplification. Alternatively, the entire variable region can be synthesized as a set of short, overlapping, oligonucleotides and combined by PCR amplification to create an entirely synthetic variable region clone. This process has certain advantages such as elimination or inclusion or particular restriction sites, or optimization of particular codons. The process can also be used to screen libraries of particular immunoglobulin encoding sequences in one species (e.g., human) to design cognate immunoglobulin encoding sequences from known antibody sequence in another species (e.g., mouse).

The nucleotide sequences of heavy and light chain transcripts from a hybridoma are used to design an overlapping set of synthetic oligonucleotides to create synthetic V sequences with identical amino acid coding capacities as the natural sequences. The synthetic heavy and kappa chain sequences can differ from the natural sequences in three ways: strings of repeated nucleotide bases are interrupted to facilitate oligonucleotide synthesis and PCR amplification; optimal translation initiation sites are incorporated according to Kozak's rules (Kozak, 1991, J. Biol. Chem.); and, restriction sites are engineered upstream of the translation initiation sites.

For both the heavy and light chain variable regions, the optimized coding, and corresponding non-coding, strand sequences are broken down into 30-50 nucleotide approximately the midpoint of the corresponding non-coding oligonucleotide. Thus, for each chain, the oligonucleotides can be assembled into overlapping double stranded sets that span segments of 150-400 nucleotides. The pools are then used as templates to produce PCR amplification products of 150-400 nucleotides. Typically, a single variable region oligonucleotide set will be broken down into two pools which are separately amplified to generate two overlapping PCR products. These overlapping products are then combined by PCR amplification to form the complete variable region. It may also be desirable to include an overlapping fragment of the heavy or light chain constant region in the PCR amplification to generate fragments that can easily be cloned into the expression vector constructs.

The reconstructed heavy and light chain variable regions are then combined with cloned promoter, leader sequence, translation initiation, leader sequence, constant region, 3' untranslated, polyadenylation, and transcription termination, sequences to form expression vector constructs. The heavy and light chain expression constructs can be combined into a single vector, co-transfected, serially transfected, or separately transfected into host cells which are then fused to form a host cell expressing both chains.

Plasmids for this use are known in the art and include the plasmids provided in the Examples section below. Similar plasmids can be constructed for expression of other heavy chain isotypes, or for expression of antibodies comprising lambda light chains. Antibodies of the present invention thus also include various isotypes (IgG, IgE, IgA, IgM, and IgD) of antibodies from different species.

In yet another aspect of the disclosure, lowered affinity antibodies can be generated by using phage and/or yeast display technology. For example, the variable regions of heavy (VH) and light chain (VL) of an antibody with known antigen specificity can be fused as a single chain Fv fragment and displayed on a phage and/or yeast surface. Displaying such scFv will exhibit strong binding activity to its target antigen. Random mutagenesis can be performed on one or more of the CDRs of the Fv Fragment, thereby constructing a phage library. The library can be selected for clones that no longer exhibit antigen binding, thereby identifying CDR mutations that eliminate or reduce antigen binding.

Validation of a reduction and/or elimination of antigen specificity of lowered affinity antibodies can be done using methods known in the art. Techniques useful for a demonstration of reduced antigen binding include, for example, FACs analysis, immunoblotting, competitive binding assays, immunohistochemistry, and surface plasmon resonance technology. Amino acids responsible for interactions between an antigen and an antibody (binding amino acids) can be identified, for example, by analysis of the interactions between the amino acids on the antigen surface and amino acids in the CDR regions of the antibody. The nature of these interactions can be classified into 3 major categories, van der Waal's forces, hydrogen bonds, and the salt bridges. Interactions are most commonly formed between antigenic determinants and CDR amino acid side chains, though hydrogen bonds may also be mediated by water molecules between antigen determinants and CDR amino acid side chains. CDR amino acids most frequently involved in Van der Waal's force interaction include Ala, Phe, Ile, Leu, Asn, Pro, Gln, Val, Trp, and Tyr. CDR amino acids that most frequently form salt bridges include Asp, Glu, Arg, and Lys. CDR amino acids most frequently involved in hydrogen bond interactions include Asp, Glu, His, Lys, Arg, Ser, and Tyr. Analysis of CDR amino acids involved in antigen interactions reveals a pattern whereby certain amino acids are more likely to be involved in antibody/antigen interactions than others. Binding amino acid/antigen interactions were determined either by site directed mutagenesis (mutational) of specific amino acids within the CDR regions, or through analysis of crystal structures of the antigen-antibody complexes (x-tal). The antibodies used in this analysis are listed in Table 1.

**Table 1. Antibodies used in antibody/antigen interaction analysis**

| **Antibody** | **Antigen** | **Method** |
|---|---|---|
| OKT3¹ | CD3 | X-tal |
| 23C3² | Osteopontin | X-tal |
| 5c8³ | CD154 | X-tal |
| 7G10⁴ | Interleukin - 23 | X-tal |
| B13I2⁵ | myohemerythrin C-helix peptide | Mutational |
| Fab4-4-20⁵ | Single strand DNA | Mutational |
| FabD1.3⁵ | Hen egg lysozyme | Mutational |
| H57⁶ | CD8 | X-tal |
| HyHEL-10⁵ | Hen egg lvsozvme | Mutational |
| HyHEL-5⁵ | Hen egg lysozyme | Mutational |
| K411B⁷ | Atrazine | Mutational |
| McPC603⁵ | Phosphocholine | Mutational |
| Rituxan⁸ | CD20 | X-tal |
| YTS156⁹ | CD8 | X-tal |

| | | |
|---|---|---|
| 1. Crystal structure of the human T cell receptor CD3 epsilon gamma heterodimer complexed to the therapeutic mAb OKT3. Kjer-Nielsen L, Dunstone MA, Kostenko L, Ely LK, Beddoe T, Mifsud NA, Purcell AW, Brooks AG, McCluskey J, Rossjohn J. (2004) Proc Natl Acad Sci U S A. 101:7675-7680. 2. Molecular basis of recognition of human osteopontin by 23C3, a potential therapeutic antibody for treatment of rheumatoid arthritis. Du J, Hou S, Zhong C, Lai Z, Yang H, Dai J, Zhang D, Wang H, Guo Y, Ding J. (2008) J Mol Biol. 382:835-842 3. Structure of CD40 ligand in complex with the Fab fragment of a neutralizing humanized antibody. Karpusas M, Lucci J, Ferrant J, Benjamin C, Taylor FR, Strauch K, Garber E, Hsu YM. (2001) Structure. 4;9:321-329. 4. Crystal structures of the pro-inflammatory cytokine interleukin-23 and its complex with a high-affinity neutralizing antibody. Beyer BM, Ingram R, Ramanathan L, Reichert P, Le HV, Madison V, Orth P. (2008) J Mol Biol. 382:942-955. 5. Structure, function and properties of antibody binding sites. Mian IS, Bradwell AR, Olson AJ. (1991) J Mol Biol. 217:133-151. 6. Atomic structure of an alphabeta T cell receptor (TCR) heterodimer in complex with an anti-TCR fab fragment derived from a mitogenic antibody. Wang J, Lim K, Smolyar A, Teng M, Liu J, Tse AG, Liu J, Hussey RE, Chishti Y, Thomson CT, Sweet RM, Nathenson SG, Chang HC, Sacchettini JC, Reinherz EL. (1998) EMBO J. 17:10-26. 7. Mapping of a hapten-binding site: molecular modeling and site-directed mutagenesis study of an anti-atrazine antibody. Kusharyoto W, Pleiss J, Bachmann TT, Schmid RD. Protein Eng. (2002) 15:233-41. 8. Structural basis for recognition of CD20 by therapeutic antibody Rituximab. Du J, Wang H, Zhong C, Peng B, Zhang M, Li B, Huo S, Guo Y, Ding J. (2007) J Biol Chem. 282:15073-80. 9. The Crystal Structure of CD8 in Complex with YTS156.7.7 Fab and Interaction with Other CD8 Antibodies Define the Binding Mode of CD8 alphabeta to MHC Class I. Shore DA, Issafras H, Landais E, Teyton L, Wilson IA. (2008) J Mol. Biol. In press | | |

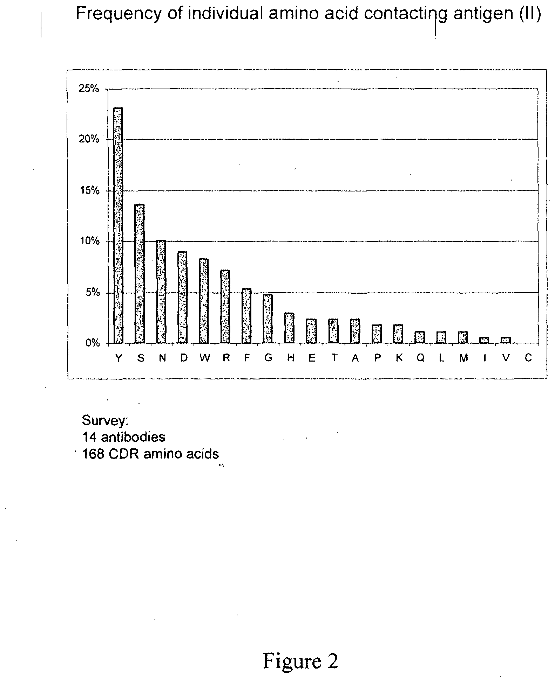

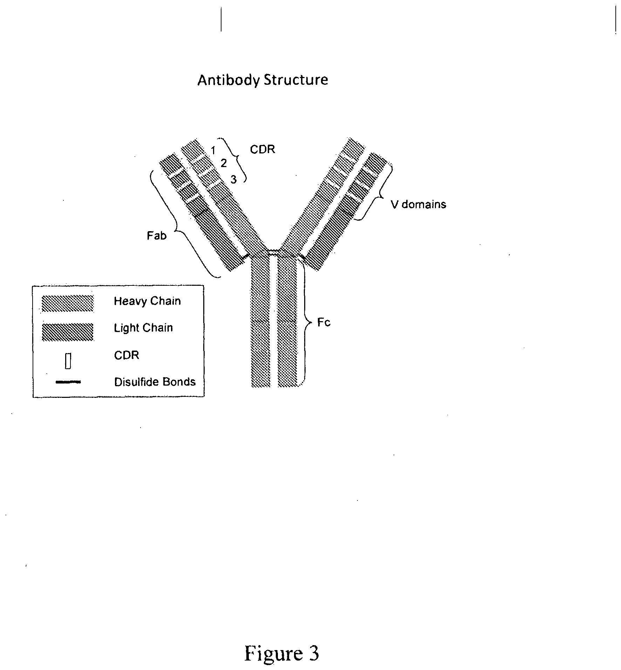

Analysis of the antibody/antigen interactions reveals that Tyr interacts with antigen determinants most frequently, accounting for about a quarter of all interactions, followed by Ser, which is responsible for about 14% of the interactions (Table 2, Figure 2). Together with Asn and Glu, these four amino acids accounted for more than half (56%) of all contacts. Such bias in antigen-contacting CDR amino acids allows the generation of an algorithm for identifying CDR amino acids that are most likely to interact with antigen determinants. Briefly, CDR regions are identified based on methods known in the art and described above. For an antibody with a known crystal structure, the structure can be analyzed to determine the CDR residues that contact antigen determinants. For an antibody whose structure is not known or available, CDR residues likely to be involved in antigen interactions can be determined based on the frequencies shown in the Table 2 and Figure 3.

Lowered affinity antibodies of the current invention can be generated by alteration of one or more amino acids involved in antibody/antigen contacts (binding amino acids) in an antibody capable of binding an antigen. Binding amino acids can be identified using any technique known in the art or described herein, including but not limited to, crystal structure analysis and use of amino acid contact frequency statistics (Table 2 and Figure 3). In some embodiments of the invention as defined in the claims, at least 1, 2, 3, 4, 5, 6, 7, 8, 9, 10, 11, 12, 13, 14, 15, 16, 17, 18, 19, 20, 21, 22, 23,24, 25,26, 27, 28, 29 or 30 binding amino acids are altered. In some embodiments, at least 10%, 25%, 50%, 60%, 70%, 80%, 85%, 90%, 95%, or 100% of the binding amino acids are altered. Alteration of binding amino acids can be performed using any method that disrupts the ability of the binding amino acid to form a contact with an antigen, including, but not limited to, replacement of the binding amino acid with a non-binding amino acid and/or deletion of the binding amino acid. Binding amino acids can also be altered through deletions, replacements, or insertions to the amino acid sequence either upstream or downstream of the binding amino acid that disrupt the ability of the binding amino acid to contact an antigen.

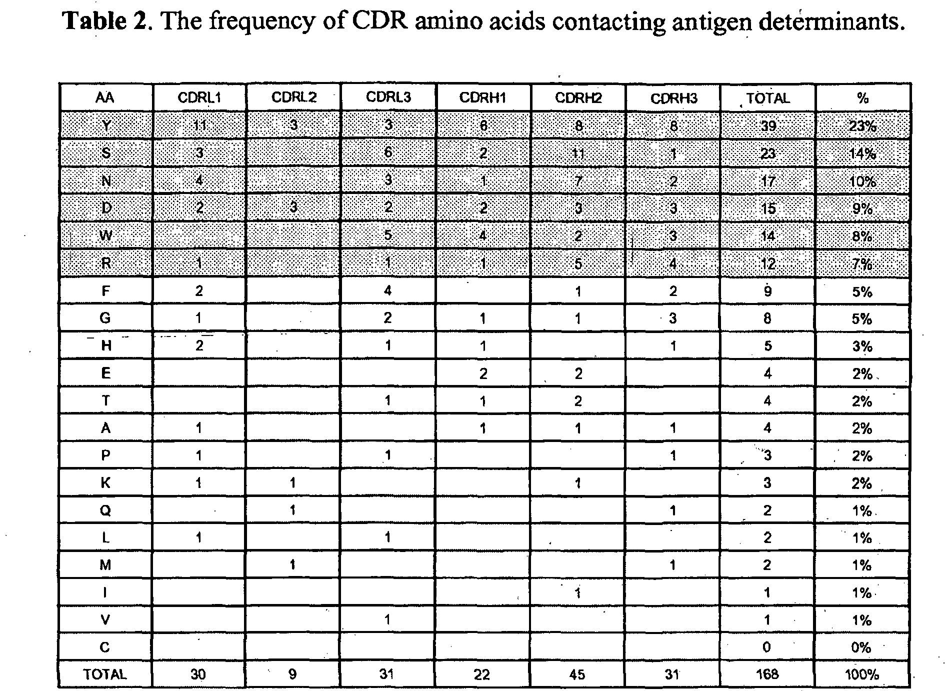

### II. Isolated Nucleic Acid Molecules

One aspect of the disclosure pertains to isolated nucleic acid molecules that encode polypeptides of the present disclosure as well as nucleic acid fragments sufficient for use as hybridization probes to identify nucleic acid molecules encoding these polypeptides and fragments for use as PCR primers for the amplification or mutation of the nucleic acid molecules. As used herein, the term "nucleic acid molecule" or "polynucleotide" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

A nucleic acid molecule can be isolated using standard molecular biology techniques and the sequence information provided herein. For example, a nucleic acid molecule can be isolated by the polymerase chain reaction (PCR) using synthetic oligonucleotide primers designed based upon the sequences of the invention.

A nucleic acid molecule can be amplified using cDNA, mRNA or, alternatively, genomic DNA as a template and appropriate oligonucleotide primers according to standard PCR amplification techniques. The nucleic acid molecule so amplified can be cloned into an appropriate vector and characterized by DNA sequence analysis. Furthermore, oligonucleotides corresponding to nucleic acid sequences of the disclosure can be prepared by standard synthetic techniques, *e.g*., using an automated DNA synthesizer.

An isolated nucleic acid molecule may comprise a nucleic acid molecule which is a complement to a described nucleic acid molecule. A nucleic acid molecule which is complementary to a described nucleic acid molecule, is one which is sufficiently complementary to a described nucleotide sequence, such that it can hybridize to the respective nucleotide sequence of the invention, thereby forming a stable duplex.

An isolated nucleic acid molecule may also comprise a nucleotide sequence which is at least about 70%, 75%, 80%, 85%, 90%, 91%, 92%, 93%, 94%, 95%, 96%, 97%, 98%, 99% or more identical to the entire length of the nucleotide sequence, or a portion of any of these nucleotide sequences.

The disclosure further encompasses nucleic acid molecules that differ from nucleotide sequence(s) that encode polypeptides of the disclosure due to degeneracy of the genetic code and thus encode the same polypeptides as those encoded by the respective nucleotide sequence. An isolated nucleic acid molecule of the disclosure has a nucleotide sequence encoding a polypeptide of the present disclosure.

Nucleic acid molecules corresponding to homologues of a nucleic acid molecule of the present disclosure can be isolated based on their homology to the nucleic acids disclosed herein using the cDNAs disclosed herein, or a portion thereof, as a hybridization probe according to standard hybridization techniques under stringent hybridization conditions.

Accordingly, an isolated nucleic acid molecule of the disclosure may be at least 15, 20, 25, 30 or more nucleotides in length and hybridizes under stringent conditions to the nucleic acid molecule comprising a nucleic acid molecule of the present disclosure.

As used herein, the term "hybridizes under stringent conditions" is intended to describe conditions for hybridization and washing under which nucleotide sequences that are significantly identical or homologous to each other remain hybridized to each other. Preferably, the conditions are such that sequences at least about 70%, more preferably at least about 80%, even more preferably at least about 85% or 90% identical to each other remain hybridized to each other. Such stringent conditions are known to those skilled in the art and can be found in Current Protocols in Molecular Biology, Ausubel et al., eds., John Wiley & Sons, Inc. (1995), sections 2, 4 and 6. Additional stringent conditions can be found in Molecular Cloning: A Laboratory Manual, Sambrook et al., Cold Spring Harbor Press, Cold Spring Harbor, N.Y. (1989), chapters 7, 9 and 11. A non-limiting example of stringent hybridization conditions includes hybridization in 4x or 6x sodium chloride/sodium citrate (SSC), at about 65-70°C (or hybridization in 4x SSC plus 50% formamide at about 42-50°C) followed by one or more washes in 1x SSC, at about 65-70°C. A further non-limiting example of stringent hybridization conditions includes hybridization at 6x SSC at 45°C, followed by one or more washes in 0.2x SSC, 0.1% SDS at 65°C. A non-limiting example of highly stringent hybridization conditions includes hybridization in 1x SSC, at about 65-70°C (or hybridization in 1x SSC plus 50% formamide at about 42-50°C) followed by one or more washes in 0.3x SSC, at about 65-70°C. A non-limiting example of reduced stringency hybridization conditions includes hybridization in 4x or 6x SSC, at about 50-60°C (or alternatively hybridization in 6x SSC plus 50% formamide at about 40-45°C) followed by one or more washes in 2x, at about 50-60°C. Ranges intermediate to the above-recited values, e.g., at 65-70°C or at 42-50°C are also intended to be encompassed by the present invention. SSPE (1x SSPE is 0.15M NaCl, 10mM NaH2PO4, and 1.25 mM EDTA, pH 7.4) can be substituted for SSC (1x SSC is 0.15M NaCl and 15mM sodium citrate) in the hybridization and wash buffers; washes are performed for 15 minutes each after hybridization is complete. The hybridization temperature for hybrids anticipated to be less than 50 base pairs in length should be 5-10°C less than the melting temperature (Tm) of the hybrid, where Tm is determined according to the following equations. For hybrids less than 18 base pairs in length, Tm (°C) =2(# of A + T bases)+4(# of G +C bases). For hybrids between 18 and 49 base pairs in length, Tm (°C) =81.5+16.6(log10[Na+])+0.41(%G+C)-(600/N), where N is the number of bases in the hybrid, and [Na+] is the concentration of sodium ions in the hybridization buffer ([Na+] for 1x SSC=0.165 M). It will also be recognized by the skilled practitioner that additional reagents may be added to hybridization and/or wash buffers to decrease non-specific hybridization of nucleic acid molecules to membranes, for example, nitrocellulose or nylon membranes, including but not limited to blocking agents (e.g., BSA or salmon or herring sperm carrier DNA), detergents (e.g., SDS), chelating agents (e.g., EDTA), Ficoll, PVP and the like. When using nylon membranes, in particular, an additional non-limiting example of stringent hybridization conditions is hybridization in 0.25-0.5M NaH2PO4, 7% SDS at about 65°C, followed by one or more washes at 0.02M NaH2PO4, 1% SDS at 65°C, see e.g., Church and Gilbert (1984) Proc. Natl. Acad. Sci. USA 81:1991-1995 (or alternatively 0.2x SSC, 1% SDS).

The skilled artisan will further appreciate that changes can be introduced by mutation into a nucleic acid molecule of the present disclosure, thereby leading to changes in the amino acid sequence of the encoded polypeptides of the present invention, without altering the functional ability of the polypeptides. For example, nucleotide substitutions leading to amino acid substitutions at "non-essential" amino acid residues can be made in a nucleic acid molecule of the present disclosure. A "non-essential" amino acid residue is a residue that can be altered from a nucleic acid molecule of the present invention without altering the biological property, whereas an "essential" amino acid residue is required for the biological property. For example, amino acid residues that are important for the structural integrity of the antibody molecules, are predicted to be particularly unamenable to alteration.

Accordingly, another aspect of the disclosure pertains to nucleic acid molecules encoding polypeptides of the present disclosure that contain changes in amino acid residues that are not essential for activity. Such polypeptides differ in amino acid sequence from those in Figures 2-7, yet retain biological activity. The isolated nucleic acid molecule may comprise a nucleotide sequence encoding a polypeptide, wherein the polypeptide comprises an amino acid sequence at least about 71%, 75%, 80%, 85%, 90%, 91%, 92%, 93%, 94%, 95%, 96%, 97%, 98%, 99% or more identical to polypeptides of the invention.

An isolated nucleic acid molecule encoding a polypeptide identical to the polypeptides of the disclosure can be created by introducing one or more nucleotide substitutions, additions or deletions into the nucleotide sequences of the invention such that one or more amino acid substitutions, additions or deletions are introduced into the encoded polypeptide. Mutations can be introduced into nucleic acid molecules of the present disclosure by standard techniques, such as site-directed mutagenesis and PCR-mediated mutagenesis. Conservative amino acid substitutions may be made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., asparagine, glutamine, serine, threonine, tyrosine, cysteine), nonpolar side chains (e.g., glycine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a polypeptide of the disclosure (e.g., those in Figures 2-7) can be replaced with another amino acid residue from the same side chain family. Alternatively, mutations can be introduced randomly along all or part of a nucleic acid molecule(s) of the present disclosure, such as by saturation mutagenesis, and the resultant mutants can be screened for biological activity to identify mutants that retain activity. Following mutagenesis of a nucleic acid molecule of the present disclosure, the encoded polypeptide can be expressed recombinantly and the activity of the polypeptide can be determined.

The expression characteristics of a nucleic acid molecules of the present disclosure within a cell line or microorganism may be modified by inserting a heterologous DNA regulatory element into the genome of a stable cell line or cloned microorganism such that the inserted regulatory element is operatively linked with the a nucleic acid molecules of the present disclosure. For example, a heterologous regulatory element may be inserted into a stable cell line or cloned microorganism, such that it is operatively linked with a nucleic acid molecules of the present invention, using techniques, such as targeted homologous recombination, which are well known to those of skill in the art, and described, e.g., in Chappel, U.S. Pat. No. 5,272,071; PCT publication No. WO 91/06667, published May 16, 1991.

### III. Isolated Polypeptide Molecules

One aspect of the disclosure pertains to isolated polypeptides. Polypeptides of the present disclosure can be isolated from cells or tissue sources by an appropriate purification scheme using standard protein purification techniques. Polypeptides of the present disclosure may be produced by recombinant DNA techniques. Alternatively, polypeptides of the present invention can be chemically synthesized using standard peptide synthesis techniques.

An "isolated" or "purified" polypeptide is substantially free of cellular material or other contaminating proteins from the cell or tissue source from which the polypeptides of the present disclosure is derived, or substantially free from chemical precursors or other chemicals when chemically synthesized. The language "substantially free of cellular material" includes preparations of polypeptide(s) of the present disclosure in which the polypeptide is separated from cellular components of the cells from which it is isolated or recombinantly produced. disclosure language "substantially free of cellular material" may include preparations of polypeptide(s) of the present disclosure having less than about 30% (by dry weight) of proteins not of the present disclosure (also referred to herein as a "contaminating protein"), more preferably less than about 20% of proteins not of the present disclosure still more preferably less than about 10% of proteins not of the present disclosure, and most preferably less than about 5% of proteins not of the present disclosure. When polypeptides of the present disclosure are recombinantly produced, it is also preferably substantially free of culture medium, i.e., culture medium represents less than about 20%, more preferably less than about 10%, and most preferably less than about 5% of the volume of the protein preparation.

The language "substantially free of chemical precursors or other chemicals" includes preparations of polypeptide(s) of the present disclosure in which the polypeptide is separated from chemical precursors or other chemicals which are involved in the synthesis of the polypeptide. In one embodiment, the language "substantially free of chemical precursors or other chemicals" includes preparations of polypeptide(s) of the present disclosure having less than about 30% (by dry weight) of chemical precursors or of proteins not of the present disclosure, more preferably less than about 20% chemical precursors or of proteins not of the present disclosure, still more preferably less than about 10% chemical precursors or of proteins not of the present disclosure, and most preferably less than about 5% chemical precursors or of proteins not of the present disclosure.

Polypeptide(s) of the present disclosure (e.g., those that encode the lowered affinity antibodies obtained by a method of the present invention) may have an amino acid sequence that includes one or more of SEQ ID NO: 1-14.

To determine the percent identity of two amino acid sequences or of two nucleic acid sequences, the sequences are aligned for optimal comparison purposes (e.g., gaps can be introduced in one or both of a first and a second amino acid or nucleic acid sequence for optimal alignment and non-identical sequences can be disregarded for comparison purposes). The length of a reference sequence aligned for comparison purposes may be at least 30%, preferably at least 40%, more preferably at least 50%, even more preferably at least 60%, and even more preferably at least 70%, 80%, 90%, 95%, 96%, 97%, 98%, 99%, 99.5%, or 99.9% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The amino acid sequences of the described polypeptide(s) will enable those of skill in the art to produce corresponding polypeptides. Such polypeptides can be produced in prokaryotic or eukaryotic host cells by expression of polynucleotides encoding a polypeptide(s) of the present disclosure. Alternatively, such polypeptides can be synthesized by chemical methods. Methods for expression of heterologous polypeptides in recombinant hosts, chemical synthesis of polypeptides, and in vitro translation are well known in the art and are described further in Maniatis et al., Molecular Cloning: A Laboratory Manual (1989), 2nd Ed., Cold Spring Harbor, N. Y.; Berger and Kimmel, Methods in Enzymology, Volume 152, Guide to Molecular Cloning Techniques (1987), Academic Press, Inc., San Diego, Calif.; Merrifield, J. (1969) J. Am. Chem. Soc. 91:501; Chaiken I. M. (1981) CRC Crit. Rev. Biochem. 11:255; Kaiser et al. (1989) Science 243:187; Merrifield, B. (1986) Science 232:342; Kent, S. B. H. (1988) Annu. Rev. Biochem. 57:957; and Offord, R. E. (1980) Semisynthetic Proteins, Wiley Publishing).

### IV. Recombinant Expression Vectors and Host Cells

Another aspect of the disclosure pertains to vectors, preferably expression vectors, containing a nucleic acid molecule encoding a polypeptide of the present disclosure (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the disclosure is intended to include such other forms of expression vectors, such as viral vectors (e.g., replication defective retroviruses, adenoviruses and adeno-associated viruses), which serve equivalent functions.

The recombinant expression vectors of the disclosure comprise a nucleic acid of the disclosure in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel (1990) Methods Enzymol. 185:3-7. Regulatory sequences include those which direct constitutive expression of a nucleotide sequence in many types of host cells and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, and the like. The expression vectors of the disclosure can be introduced into host cells to thereby produce proteins or peptides, including fusion proteins or peptides, encoded by nucleic acids as described herein.

The recombinant expression vectors of the disclosure can be designed for expression of polypeptides of the present disclosure in prokaryotic or eukaryotic cells. For example, the polypeptides can be expressed in bacterial cells such as E. coli, insect cells (using baculovirus expression vectors), yeast cells, or mammalian cells. Suitable host cells are discussed further in Goeddel (1990) supra. Alternatively, the recombinant expression vector can be transcribed and translated in vitro, for example using T7 promoter regulatory sequences and T7 polymerase.

Expression of polypeptides in prokaryotes is most often carried out in E. coli with vectors containing constitutive or inducible promoters directing the expression of either fusion or non-fusion proteins. Fusion vectors add a number of amino acids to a polypeptide encoded therein, usually to the amino terminus of the recombinant polypeptide. Such fusion vectors typically serve three purposes: 1) to increase expression of recombinant polypeptide; 2) to increase the solubility of the recombinant polypeptide; and 3) to aid in the purification of the recombinant polypeptide by acting as a ligand in affinity purification. Often, in fusion expression vectors, a proteolytic cleavage site is introduced at the junction of the fusion moiety and the recombinant polypeptide to enable separation of the recombinant polypeptide from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D. B. and Johnson, K. S. (1988) Gene 67:31-40), pMAL (New England Biolabs, Beverly, Mass.) and pRIT5 (Pharmacia, Piscataway, NJ) which fuse glutathione S-transferase (GST), maltose E binding protein, or protein A, respectively, to the target recombinant polypeptide.

Examples of suitable inducible non-fusion E. coli expression vectors include pTrc (Amann et al. (1988) Gene 69:301-315) and pET 1 Id (Studier et al. (1990) Methods Enzymol. 185:60-89). Target gene expression from the pTrc vector relies on host RNA polymerase transcription from a hybrid trp-lac fusion promoter. Target gene expression from the pET 11 d vector relies on transcription from a T7 gn10-lac fusion promoter mediated by a coexpressed viral RNA polymerase (T7 gn1). This viral polymerase is supplied by host strains BL21(DE3) or HMS174(DE3) from a resident prophage harboring a T7 gn1 gene under the transcriptional control of the lacUV 5 promoter.

One strategy to maximize recombinant polypeptide expression in E. coli is to express the polypeptide in host bacteria with impaired capacity to proteolytically cleave the recombinant polypeptide (Gottesman, S. (1990) Methods Enzymol. 185:119-128). Another strategy is to alter the nucleic acid sequence of the nucleic acid to be inserted into an expression vector so that the individual codons for each amino acid are those preferentially utilized in E. coli (Wada et al. (1992) Nucleic Acids Res. 20:2111-2118). Such alteration of nucleic acid sequences of the disclosure can be carried out by standard DNA synthesis techniques.

The expression vector may be a yeast expression vector. Examples of vectors for expression in yeast S. cerevisiae include pYepSec1 (Baldari et al. (1987) EMBO J. 6:229-234), pMFa (Kurjan and Herskowitz (1982) Cell 30:933-943), pJRY88 (Schultz et al. (1987) Gene 54:113-123), pYES2 (Invitrogen Corporation, San Diego, Calif.), and picZ (Invitrogen Corp, San Diego, Calif.).

Alternatively, polypeptides of the present disclosure (e.g., Figures 2-7) can be expressed in insect cells using baculovirus expression vectors. Baculovirus vectors available for expression of polypeptides in cultured insect cells (e.g., Sf 9 cells) include the pAc 'series (Smith et al. (1983) Mol. Cell Biol. 3:2156-2165) and the pVL series (Lucklow and Summers (1989) Virology 170:31-39).

A nucleic acid of the present disclosure (e.g., Figures 2-7) may be expressed in mammalian cells using a mammalian expression vector. Examples of mammalian expression vectors include pCDM8 (Seed, B. (1987) Nature 329:840) and pMT2PC (Kaufman et al. (1987) EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J. et al., Molecular Cloning: A Laboratory Manual. 2nd ed., Cold Spring Harbor Laboratory, Cold Spring Harbor Laboratory Press, Cold Spring Harbor, N.Y., 1989.

The recombinant mammalian expression vector may be capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), particular promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters (e.g., the neurofilament promoter; Byrne and Ruddle (1989) Proc. Natl. Acad. Sci. USA 86:5473-5477), pancreas-specific promoters (Edlund et al. (1985) Science 230:912-916), and mammary gland-specific promoters (e.g., milk whey promoter; U.S. Pat. No. 4,873,316 and European Application Publication No. 264,166). Developmentally-regulated promoters are also encompassed, for example by the murine hox promoters (Kessel and Gruss (1990) Science 249:374-379) and the .alpha.-fetoprotein promoter (Campes and Tilghman (1989) Genes Dev. 3:537-546).

Another aspect of the disclosure pertains to host cells into which a nucleic acid molecule of the present disclosure is introduced within a recombinant expression vector or a nucleic acid molecule containing sequences which allow it to homologously recombine into a specific site of the host cell's genome. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a polypeptide of the present disclosure can be expressed in bacterial cells such as E. coli, insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation. Suitable methods for transforming or transfecting host cells can be found in Sambrook et al. (Molecular Cloning: A Laboratory Manual. 2nd, ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as the polynucleotide of the invention or can be introduced on a separate vector. Cells stably transfected with the introduced nucleic acid can be identified by drug selection (e.g., cells that have incorporated the selectable marker gene will survive, while the other cells die).

A host cell of the disclosure such as a prokaryotic or eukaryotic host cell in culture, can be used to produce (i.e., express) a polypeptide of the present disclosure. Accordingly, the disclosure further provides methods for producing a polypeptide of the present disclosure using the host cells of the present disclosure. The may comprise culturing the host cell of the disclosure (into which a recombinant expression vector encoding a polypeptide of the present disclosure has been introduced) in a suitable medium such that a polypeptide of the present disclosure is produced. The method may further comprise isolating a polypeptide of the present disclosure from the medium or the host cell.

The host cells of the disclosure can also be used to produce non-human transgenic animals, as described below.

### V. Methods of Use

Lowered affinity antibodies can be used in assays designated to diagnostically or prognostically monitor polypeptide levels in tissue as part of a clinical testing procedure, e.g., to, for example, determine the efficacy of a given treatment regimen. Detection can be facilitated by coupling (i e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, P-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include 125I, 131I, 35S or 3H.

### Example 1 Generation and Characterization of Novel Lowered Affinity Antibodies

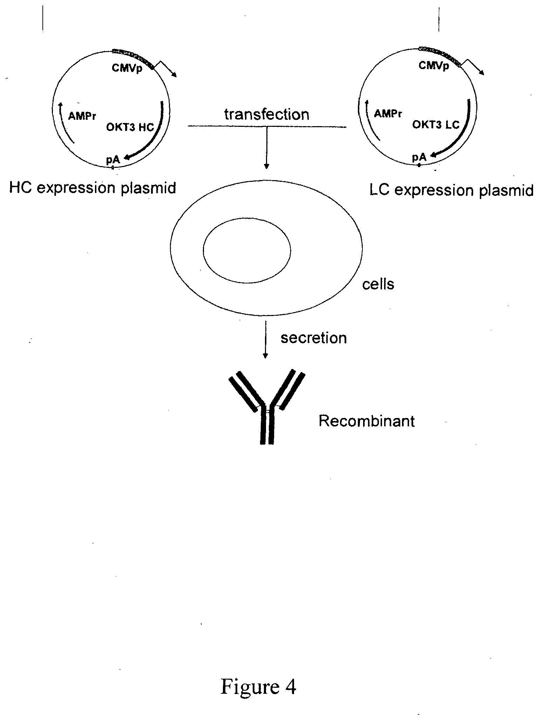

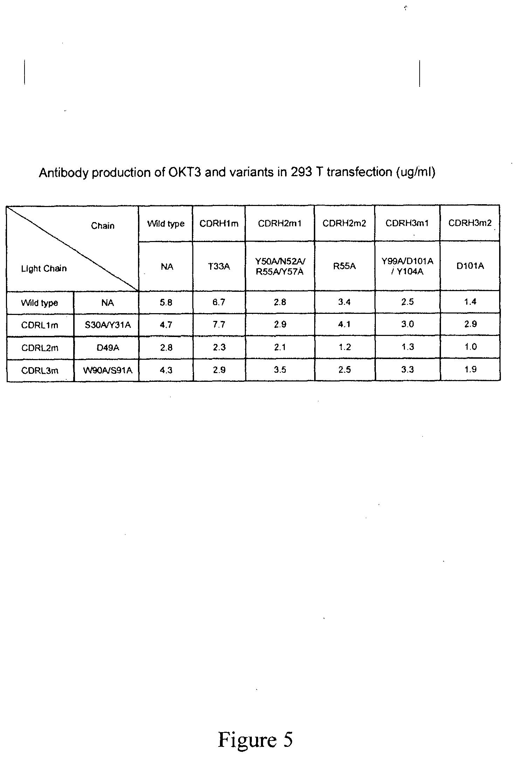

The amino acid sequences of OKT3 complementarity determining regions were modified to reduce or eliminate antigen binding without affecting antibody structure or expression. Exemplary CDR sequences of control antibodies of the invention are listed in Table 3. The resulting lowered affinity antibodies expressed normally (Figure 3), and had minimal or no capacity to bind to human Jurkat cells (Figure 4) or human peripheral blood mononuclear cells (PBMCs, Figure 5).

**Table 3. Exemplary lowered affinity antibody CDR sequences.**

| CDR | Substitution | Sequence | SEQ ID NO. |
|---|---|---|---|
| OKT3 CDRH1 | NA | GYTFTRYTMH | SEQ ID NO: 1 |
| CDRH1m | T33A | GYTFTRYAMH | SEQ ID NO: 2 |
| OKT3 CDRH2 | NA | YINPSRGYTNYNQKFKD | SEQ ID NO: 3 |
| CDRH2m1 | Y50A/N52A/R55A/Y57A | AIAPSAGATNYNQKFKD | SEQ ID NO: 4 |
| CDRH2m2 | R55A | YINPSAGYTNYNQKFKD | SEQ ID NO: 5 |
| OKT3 CDRH3 | NA | YYDDHYCLDY | SEQ ID NO: 6 |
| CDRH3m1 | Y99A/D101A/Y104A | AYADHACLDY | SEQ ID NO: 7 |
| CDRH3m2 | D101A | YYADHYCLDY | SEQ ID NO: 8 |
| OKT3 CDRL1 | NA | SASSSVSY | SEQ ID NO: 9 |
| CDRL1m | S30A/Y31A | SASSSVAA | SEQ ID NO: 10 |
| OKT3 CDRL2 | NA | IYDTSKL | SEQ ID NO: 11 |
| CDRL2m | D49A | IYATSKL | SEQ ID NO: 12 |
| OKT3 CDRL3 | NA | QQWSSNPFT | SEQ ID NO: 13 |
| CDRL3m | W90A/S91A | QQAASNPFT | SEQ ID NO: 14 |

### Cell lines and Media

Human 293T cells were maintained in Dulbecco's Modified Eagles's medium (DMEM) supplemented with 10% fetal bovine serum (FBS) (Invitogen, Gibco, Carlsbad, CA), 2 mM L-glutamine, and 40 ug/ml of gentamicin. Mouse OKT3, an anti-human CD3 antibody producing hybridoma cell line, was grown in Iscove's Modified Dulbecco's medium (IMDM) containing 10% FBS, 1mM sodium pyruvate, 2 mM L-glutamine, and 40 ug/ml of gentamicin. Jurkat E6.1, a human leukemic T cell line, was grown in RPMI medium supplemented with 10% FBS, 2 mM L-glutamine, and 40 ug/ml of gentamicin. All media and supplements were purchased from Lonza, Walkville, MD, except for FBS.

### Construction of OKT3 heavy and light chain expression plasmids

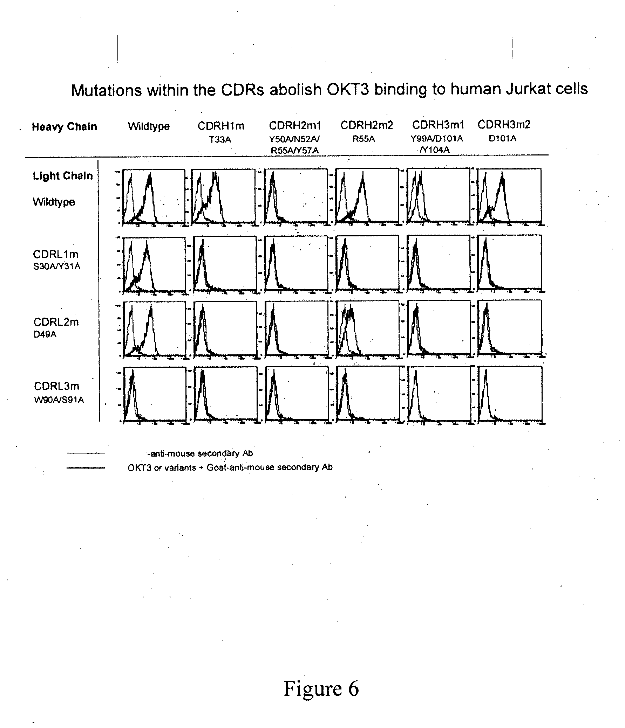

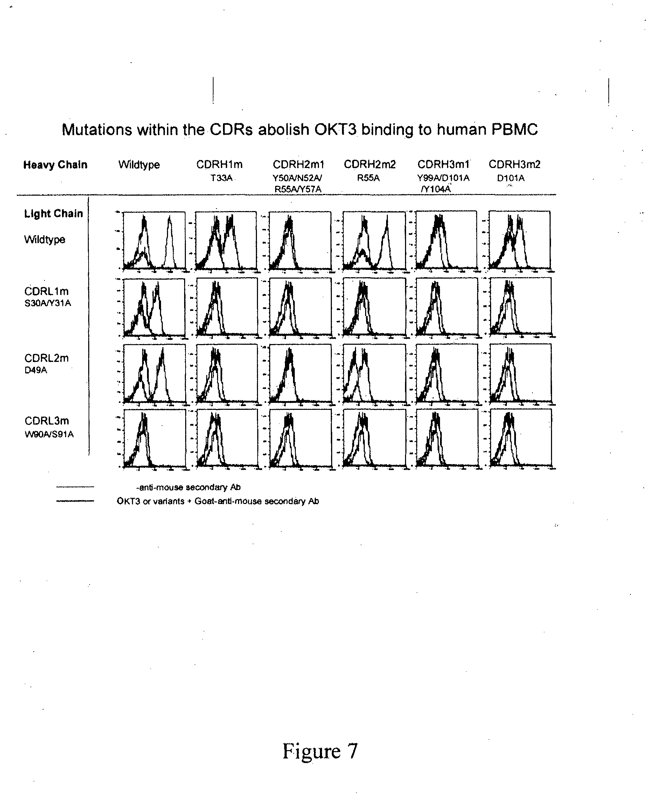

Total RNA was isolated from OKT3 hybridoma cell line using TRIzol Reagent from Invitrogen. cDNA was synthesized using SuperScript™ III Reverse Transcriptase (Invitrogen) and Oligo (dT)12-18 Primer (Invitrogen). Based on Genbank records of mouse OKT3 heavy chain (Accession #A22261) and light chain (Accession #A22259) nucleotide sequences, the entire coding sequences of the heavy and light chains were amplified via polymerase chain reactions (PCR) with oligo dT-primed cDNA of OKT3 hybridoma as the template, and cloned into a mammalian expression plasmid, driven by CMV Immediate Early gene promoter, called pME. The complete nucleotide sequence of plasmid pME-wtOKT3 HC (Figure 6) is represented in SEQ ID NO: 23 and the complete nucleotide sequence of plasmid pME-wtOKT3LC (Figure 7) is represented in SEQ ID NO: 24.

### Construction of expression plasmids encoding OKT3 heavy and light chain variants

Site directed mutagenesis was performed using standard PCR techniques to generate the OKT3 heavy and light chain variants, replacing various antigen contacting amino acids within the CDRs with alanine. PCR fragments harboring the mutations were cloned into the same expression plasmid pME. The resultant plasmids were confirmed by sequencing reactions.

### Transfection

The OKT3 and variant antibodies were produced by transient transfection using the standard calcium phosphate method. Briefly, 293T cells were seeded in 6-well plates at 6 x 10⁵ cells/3 ml 293T medium/well 20-24 hours before transfection. 3 hours prior to transfection, the culture medium was changed to IMDM supplemented with 10% FBS, 25mM Hepes buffer and 40 ug/ml gentamycin. The transfection procedure was carried out by first mixing 8 µg each of the OKT3 heavy and light chain plasmid DNAs with 31 µl of 2M CaCl₂ and sterile distilled water for a final volume of 250 µl. The DNA/calcium mixture was then slowly added to 250 µl of 2x HBS buffer (281 mM NaCl, 100 mM HEPES, 1.5mM Na₂HPO₄ at pH 7.12). The transfection mixture was incubated at room temperature for 20 minutes, and then added slowly to the 293T cultures. 12-16 hours after transfection, the culture medium was changed once again to the complete 293T medium. 40-48 hours after transfection, culture supernatants containing OKT3 variant antibodies were harvested, and used for subsequent analyses.

### Antibody quantitation

The amount of OKT3 and variant antibodies produced by transient transfection was determined by the Guava RapidQuant mouse IgG Kit (Guava Technologies, Hayward, CA) following the manufacture's protocol. In brief, 10 µl of supernatant from each transfection was incubated with IgG capture beads for 40 min with shaking. Subsequently, FITC-conjugated goat anti-mouse IgG was added to each sample and incubated 60 minutes with shaking. The sample volume was brought up to 200 µl and run on Guava EasyCyte system (Guava). The antibody concentration in each sample was calculated by comparing the mean florescence channel (MFI) of the samples with those of the standards.

### Isolation of human peripheral mononuclear cells (hPBMC)

Human peripheral mononuclear cells (hPBMC) were isolated using Ficoll-paque gradient method. In brief, human whole blood was diluted 1:1 with 1x Phosphate Buffered Saline (PBS) and carefully layered on the top of Ficoll-paque (0.4 volume of blood sample volume) (GE Healthcare, Uppsala, Sweden). After centrifugation at 900 g for 30 min, hPBMC at the interface of blood and Ficoll-paque were carefully extracted. Cell suspensions were diluted 1:3 with 1x PBS and spun at 400 g for 10 min at 8-20°C. Cell pellets were washed one more time with 1x PBS at 400 g for 10 min at 8-20°C and diluted with FACS buffer (PBS with 2% FCS, 0.1% NaN₃) for flow cytometric analysis.

### Flow cytometry

Human Jurkat cells (1 x 10⁵ cells/well in 96-well plates) or hPBMC (3 x 10⁵ cells/well in 96-well plates) were incubated with 100 µl of transfection supernatant of OKT3 and variants at 4°C for 60 minute and washed twice with 200 µl of FACS buffer. Cells were stained with 100 µl of phycoerythrin (PE)-conjugated goat-anti-mouse Immunoglobulin G (Invitrogen-Caltag, Carlsbad, CA) for 60 min at 4°C, and washed twice with 200 ul of FACS buffer. The cells were then fixed with 1% paraformaldehyde for 15 minute at 4°C, and washed once with FACS buffer. Cells were then resuspended in 200 µl of FACS buffer for analysis on a Guava EaseCyte Plus system (Guava Technologies, Inc., Hayward, CA).

### Binding of OKT3 and OKT3 variant to Jurkat cells.

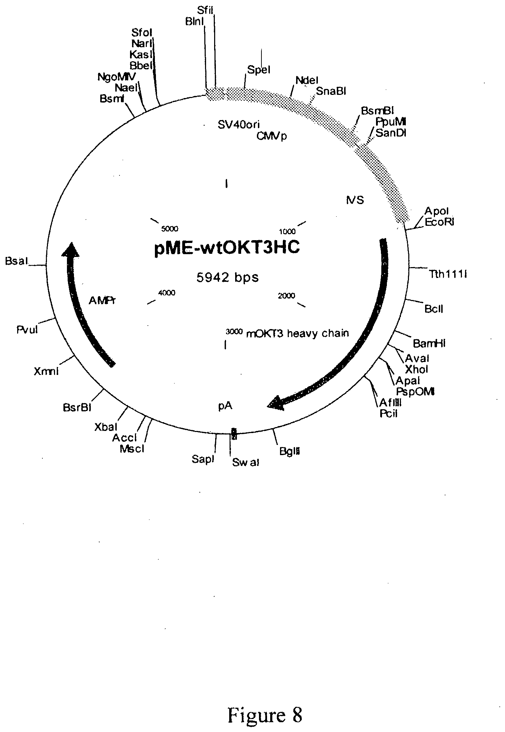

A total of 1x10⁵ Jurket cells were incubated with serially diluted OKT3 or OKT3 variant antibody, followed by PE-conjugated goat anti-mouse IgG, and analyzed by the FACS. At 1600 ng/ml, binding of OKT3 variant is comparable to that of OKT3 antibody at 0.4 ng/ml. Thus, the binding affinity of OKT3 variant is approximately 4000 x less than that of the parental OKT3 antibody (Figure 8).

### Pharmacokinetics of the variant antibody.

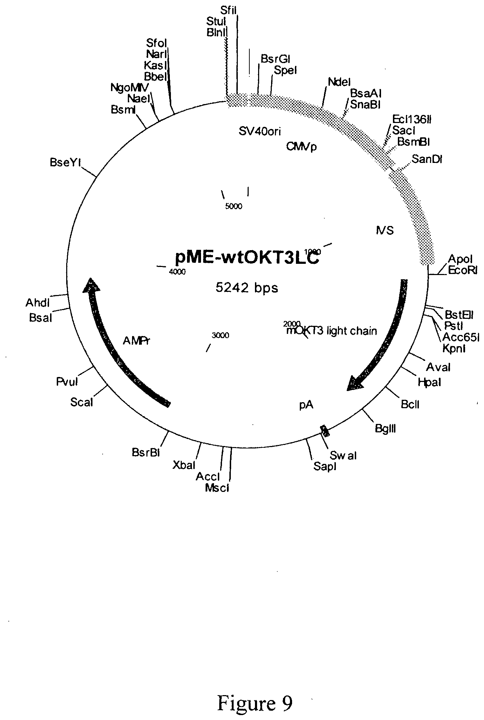

Pharmacokinetic analysis of the variant IgG2a antibody. An IgG2a antibody was produced by co-expressing of a CDR mutated heavy chain (Seq No. 26) and a CDR mutated light chain (Seq No. 47). While it is clear that this variant antibody no longer binds to the CD3 which is the antigen of the parental antibody, OKT3, it is not known whether this antibody may serendipitously interact yet with another antigen(s). One way to test this is to determine if the variant antibody has *an in vivo* half life similar to endogenous circulating IgG2a antibody. The variant antibody was administered in B6 mice and serum samples were collected up to 21 days. The concentration of recombinant IgG2a antibody was measured by an allotype specific anti-mouse antibody. The half life of the antibody was calculated by fitting the beta-phase of the pharmacokinetic profile and determined to be 13.75 days (Figure 9). This half life is in close agreement with the reported 10-15 days half life of murine IgG2a antibody (Talbotp, P. J. and Buchmeie M. J. Catabolism of homologous murine monoclonal hybridoma IgG antibodies in mice. Immunology 60:485-489, 1987). The long serum half life indicates that this antibody is not likely to interact with an unexpected antigen(s) and the *in vivo* system is not able to discriminate this variant antibody from the endogenous immunoglobulin of the same isotype.

## Claims

1. A method for producing a recombinant antibody with reduced binding affinity, comprising the steps of:
a) identifying by contact frequency statistics at least one amino acid from within a complementary determining region (CDR) of an antibody which is capable of a binding interaction with a target antigen;
b) altering the amino acid sequence of the CDR by replacing at least one interacting amino acid with a non-interacting amino acid without altering the overall antibody structure; and
c) confirming the binding interactions with the antigen arid the recombinant antibody are reduced or eliminated such that the recombinant antibody has an approximate dissociation constant (KD) of greater than or equal to about 10⁻⁷ M.

2. The method of claim 1, further comprising
d) confirming the recombinant antibody has an *in vivo* half-life similar to endogenous circulating immunoglobulins.

3. The method of any of claims 1 and 2, wherein the identified amino acid in the CDR polypeptide sequence is selected from the group consisting of tyrosine, serine, asparagine, aspartic acid, tryptophan and arginine.

4. The method of any of claims 1 and 2, wherein said non- interacting amino acid is selected from the group consisting of alanine, valine, leucine, isoleucine, proline and methionine.

5. The method of claim 4, wherein said non- interacting amino acid is alanine.

6. The method of claim 1, wherein the step of identifying the interacting amino acid further includes analyzing a crystal structure depicting a complex between the antibody, antibody fragment, or the CDR polypeptide sequence and the antigen.

7. The method of claim 1, further comprising the step of validating a loss of antigen binding.

8. The method of claim 7, wherein the step of validation is accomplished by FACS, ELISA, or radioimmunoassay.

9. The method of any of claims 1 to 8, further comprising expressing the recombinant antibody in a mammalian cell system.

## Patentansprüche

1. Verfahren zur Herstellung eines rekombinanten Antikörpers mit verringerter Bindungsaffinität, umfassend die Schritte:
a) Identifizieren mittels Kontaktfrequenzstatistiken wenigstens einer Aminosäure aus dem Inneren einer komplementaritätsbestimmenden Region (CDR) eines Antikörpers, der zu einer bindenden Wechselwirkung mit einem Zielantigen in der Lage ist;
b) Ändern der Aminosäuresequenz der CDR durch Ersetzen wenigstens einer wechselwirkenden Aminosäure durch eine nicht-bindende Aminosäure, ohne die Gesamtstruktur des Antikörpers zu ändern;
c) Bestätigen, dass die bindenden Wechselwirkungen mit dem Antigen und dem rekombinanten Antikörper verringert oder beseitigt sind, so dass der rekombinante Antikörper eine ungefähre Dissoziationskonstante (KD) von größer als oder gleich etwa 10⁻⁷ M aufweist.

2. Verfahren nach Anspruch 1, weiter umfassend
d) Bestätigen, dass der rekombinante Antikörper eine *in vivo* Halbwertszeit ähnlich endogenen zirkulierenden Immunglobulinen aufweist.

3. Verfahren nach einem der Ansprüche 1 und 2, wobei die identifizierte Aminosäure in der CDR-Polypeptid-Sequenz ausgewählt ist aus der Gruppe bestehend aus Tyrosin, Serin, Asparagin, Asparaginsäure, Tryptophan und Arginin.

4. Verfahren nach einem der Ansprüche 1 und 2, wobei die nicht-wechselwirkende Aminosäure ausgewählt ist aus der Gruppe bestehend auf Alanin, Valin, Leucin, Isoleucin, Prolin und Methionin.

5. Verfahren nach Anspruch 4, wobei die nicht-wechselwirkende Aminosäure Alanin ist.

6. Verfahren nach Anspruch 1, wobei der Schritt des Identifizierens der wechselwirkenden Aminosäure weiter umfasst Analysieren einer Kristallstruktur, die einen Komplex zwischen dem Antikörper, dem Antikörperfragment oder der CDR-Polypeptid-Sequenz und dem Antigen darstellt.

7. Verfahren nach Anspruch 1, weiter umfassend den Schritt des Validierens eines Verlusts an Antigenbindung.

8. Verfahren nach Anspruch 7, wobei der Schritt des Validierens durch FACS, ELISA oder Radioimmunoassay erfolgt.

9. Verfahren nach einem der Ansprüche 1 bis 8, weiter umfassend Expression des rekombinanten Antikörpers in einem Säugetierzellsystem.

## Revendications

1. Procédé de production d'un anticorps recombinant à affinité de liaison réduite, comprenant les étapes :
a) d'identification par des statistiques de fréquence des contacts d'au moins un acide aminé appartenant à une région de détermination de la complémentarité (RDC) d'un anticorps qui est capable de réaliser une interaction de liaison avec un antigène cible ;
b) d'altération de la séquence d'acides aminés de la RDC en remplaçant au moins un acide aminé qui est en interaction par un acide aminé qui n'est pas en interaction sans altérer la structure globale de l'anticorps ; et
c) de confirmation de la réduction ou de l'élimination des interactions de liaison avec l'antigène et l'anticorps recombinant de sorte que l'anticorps recombinant présente une constante de dissociation (KD) approximative supérieure ou égale à environ 10⁻⁷ M.

2. Procédé selon la revendication 1, comprenant en outre
d) de confirmation que l'anticorps recombinant a une demi-vie *in vivo* similaire à celle des immunoglobulines circulantes endogènes.

3. Procédé selon l'une quelconque des revendications 1 et 2, dans lequel l'acide aminé identifié dans la séquence polypeptidique de la RDC est choisi dans le groupe constitué par une tyrosine, une sérine, une asparagine, un acide aspartique, un tryptophane et une arginine.

4. Procédé selon l'une quelconque des revendications 1 et 2, dans lequel ledit acide aminé qui n'est pas en interaction est choisi dans le groupe constitué par une alanine, une valine, une leucine, une isoleucine, une proline et une méthionine.

5. Procédé selon la revendication 4, dans lequel ledit acide aminé qui n'est pas en interaction est une alanine.

6. Procédé selon la revendication 1, dans lequel l'étape d'identification de l'acide aminé qui est en interaction comprend en outre l'analyse d'une structure cristalline représentant un complexe entre l'anticorps, le fragment d'anticorps, ou la séquence polypeptidique de la RDC et l'antigène.

7. Procédé selon la revendication 1, comprenant en outre l'étape de validation d'une perte de liaison à l'antigène.

8. Procédé selon la revendication 7, dans lequel l'étape de validation est accomplie par FACS, ELISA, ou un dosage radio-immunologique.

9. Procédé selon l'une quelconque des revendications 1 à 8, comprenant en outre l'expression de l'anticorps recombinant dans un système de cellules de mammifères.


### SEQUENCE LISTING

<110> AB Biosciences, Inc.
<120> NOVEL LOWERED AFFINITY ANTIBODIES AND METHODS OF MAKING THE SAME
<130> A 10039 EP
<140> EP 11 737 760.6
   <141> 2011-01-28
<150> 61/299,162
   <151> 2010-01-28
<160> 14
<170> PatentIn version 3.5
<210> 1
   <211> 10
   <212> PRT
   <213> Artificial Sequence
<220>
   <223> Description of Artificial Sequence: Synthetic peptide
<400> 1
   Gly Tyr Thr Phe Thr Arg Tyr Thr Met His
   1               5                   10
<210> 2
   <211> 10
   <212> PRT
   <213> Artificial Sequence
<220>
   <223> Description of Artificial Sequence: Synthetic peptide
<400> 2
   Gly Tyr Thr Phe Thr Arg Tyr Ala Met His
   1               5                   10
<210> 3
   <211> 17
   <212> PRT
   <213> Artificial Sequence
<220>
   <223> Description of Artificial Sequence: Synthetic peptide
<400> 3
   Tyr Ile Asn Pro Ser Arg Gly Tyr Thr Asn Tyr Asn Gln Lys Phe Lys
   1               5                   10                  15
   Asp
<210> 4
   <211> 17
   <212> PRT
   <213> Artificial Sequence
<220>
   <223> Description of Artificial Sequence: Synthetic peptide
<400> 4
   Ala Ile Ala Pro Ser Ala Gly Ala Thr Asn Tyr Asn Gln Lys Phe Lys
   1               5                   10                  15
   Asp
<210> 5
   <211> 17
   <212> PRT
   <213> Artificial Sequence
<220>
   <223> Description of Artificial Sequence: Synthetic peptide
<400> 5
   Tyr Ile Asn Pro Ser Ala Gly Tyr Thr Asn Tyr Asn Gln Lys Phe Lys
   1               5                   10                  15
   Asp
<210> 6
   <211> 10
   <212> PRT
   <213> Artificial Sequence
<220>
   <223> Description of Artificial Sequence: Synthetic peptide
<400> 6
   Tyr Tyr Asp Asp His Tyr Cys Leu Asp Tyr
   1               5                   10
<210> 7
   <211> 10
   <212> PRT
   <213> Artificial Sequence
<220>
   <223> Description of Artificial Sequence: Synthetic peptide
<400> 7
   Ala Tyr Ala Asp His Ala Cys Leu Asp Tyr
   1               5                   10
<210> 8
   <211> 10
   <212> PRT
   <213> Artificial Sequence
<220>
   <223> Description of Artificial Sequence: Synthetic peptide
<400> 8
   Tyr Tyr Ala Asp His Tyr Cys Leu Asp Tyr
   1               5                   10
<210> 9
   <211> 8
   <212> PRT
   <213> Artificial Sequence
<220>
   <223> Description of Artificial Sequence: Synthetic peptide
<400> 9
   Ser Ala Ser Ser Ser Val Ser Tyr
   1               5
<210> 10
   <211> 8
   <212> PRT
   <213> Artificial Sequence
<220>
   <223> Description of Artificial Sequence: Synthetic peptide
<400> 10
   Ser Ala Ser Ser Ser Val Ala Ala
   1               5
<210> 11
   <211> 7
   <212> PRT
   <213> Artificial Sequence
<220>
   <223> Description of Artificial Sequence: Synthetic peptide
<400> 11
   Ile Tyr Asp Thr Ser Lys Leu
   1               5
<210> 12
   <211> 7
   <212> PRT
   <213> Artificial Sequence
<220>
   <223> Description of Artificial Sequence: Synthetic peptide
<400> 12
   Ile Tyr Ala Thr Ser Lys Leu
   1               5
<210> 13
   <211> 9
   <212> PRT
   <213> Artificial Sequence
<220>
   <223> Description of Artificial Sequence: Synthetic peptide
<400> 13
   Gln Gln Trp Ser Ser Asn Pro Phe Thr
   1               5
<210> 14
   <211> 9
   <212> PRT
   <213> Artificial Sequence
<220>
   <223> Description of Artificial Sequence: Synthetic peptide
<400> 14
   Gln Gln Ala Ala Ser Asn Pro Phe Thr
   1               5